Europäisches Patentamt

(19) European Patent Office

Office européen des brevets

(11) EP 0 633 780 B1

## (12) FASCICULE DE BREVET EUROPEEN

(45) Date de publication et mention
de la délivrance du brevet:
**05.06.1996 Bulletin 1996/23**

(21) Numéro de dépôt: **93907904.2**

(22) Date de dépôt: **29.03.1993**

(51) Int. Cl.[6]: **A61K 31/54**

(86) Numéro de dépôt international:
**PCT/FR93/00312**

(87) Numéro de publication internationale:
**WO 93/19757 (14.10.1993 Gazette 1993/25)**

(54) **EMPLOI DES DERIVES DE LA PHENOTHIAZINE DANS LE TRAITEMENT DE L'ISCHEMIE ET/OU DE L'HYPOXIE**

VERWENDUNG VON PHENOTHIAZIN-DERIVATEN ZUR BEHANDLUNG DER ISCHÄMIE UND/ODER DER HYPOXÄMIE

USE OF PHENOTHIAZINE DERIVATIVES FOR TREATING ISCHAEMIA AND/OR HYPOXIA

(84) Etats contractants désignés:
**AT BE CH DE DK ES FR GB GR IE IT LI LU NL PT SE**

(30) Priorité: **30.03.1992 FR 9203793**

(43) Date de publication de la demande:
**18.01.1995 Bulletin 1995/03**

(73) Titulaire: **RHONE-POULENC RORER S.A.**
**92160 Antony (FR)**

(72) Inventeurs:
- **GARRET, Claude**
  **F-94120 Fontenay-sous-Bois (FR)**
- **RATAUD, Jean**
  **F-95600 Eaubonne (FR)**
- **STUTZMANN, Jean-Marie**
  **F-94440 Villecresnes (FR)**

(74) Mandataire: **Savina, Jacques et al**
**Rhône-Poulenc Rorer S.A.**
**Direction Brevets (t144)**
**20 avenue Raymond Aron**
**F-92165 Antony Cédex (FR)**

(56) Documents cités:
EP-A- 0 419 360          US-A- 3 112 310
US-A- 4 833 138          US-A- 5 049 669

- **ANN.IST.SUPER.SANITA' vol. 7, 1971, pages 601 - 4 'Effetto preventivo di composti fenotiazinici e tioxantenici sulla necrosi epatica sperimantale da ischemia'**
- **J.MOL.CELL.CARDIOL. vol. 15, no. 9, 1983, pages 621 - 8 'Chloropromazine Inhibits Loss of Contractile Function, Compliance and ATP in Ischemic Rabbit Heart'**
- **HISTOCHEM.J. vol. 16, 1984, pages 383 - 4 'Effect of Chloropromazine on the Extent of Ischemic Changes of the Myocardium. A Histochemical Study'**

Remarques:
Le dossier contient des informations techniques présentées postérieurement au dépôt de la demande et ne figurant pas dans le présent fascicule.

**Description**

La présente invention concerne une nouvelle application thérapeutique des dérivés de la phénothiazine, choisis parmi le N-n-propyl-[pyrrolidinyl-1)-1 propyl-2]-10 phénothiazinecarboxamide-2 (série L) et le N-(méthyl-2 butyl) [(pyrrolidinyl-1)-1 propyl-2]-10 phénothiazinecarboxamide-2 (série L) et leurs sels.

Ces deux derivés sont connus pour leur activité analgésique diurétique ainsi que pour leur activité sur le système nerveux central : US 5 049 669 et US 3 112 310

Il a été maintenant trouvé que ces dérivés de la phénothiazine, ainsi que leurs sels, possèdent une activité dans le traitement de l'hypoxie et/ou de l'ischémie.

Des dérivés de la phénothiazine utiles dans le traitement de l'ischémie sont décrits dans le brevet US 4 833 138 et dans les articles parus dans J. Mol. Cell. Cardiol., 1983, 15, p. 621-8 et Histochemical Journal, 1984, 16, p. 383-384.

L'activité des produits a pu être mise en évidence dans la méthode des crises induites par l'acide N-méthyl-DL-aspartique (NMDLA).

Il existe en effet de nombreuses données expérimentales qui indiquent que le glutamate neuromédiateur excitateur est un neurotoxique et que le complexe récepteur-glutamate N-méthyl D-aspartate (NMDA) (l'un des récepteurs glutamatergiques) est impliqué dans la pathologie de l'ischémie cérébrale.

Il est bien démontré que les antagonistes NMDA manifestent une activité protectrice vis-à-vis des dommages provoqués par l'hypoxie ou l'ischémie cérébrale : U. Dirnagl, J. Tanabe, et W. Pulsinelli, Brain Reseach, 527, 62 (1990).

Les deux dérivés de la phénothiazine, ainsi que leurs sels, sont tout à fait appropriés pour la préparation d'un médicament destiné au traitement préventif et/ou curatif de l'ischémie et/ou de l'hypoxie, et notamment dans les indications suivantes :

- accidents vasculaires cérébral, hémorragique ou embolique ;
- hémorragie sous-arachnoidïennes ;
- traumatisme crânien avec souffrance cérébrale ;
- état de mal épileptique avec souffrance cérébrale ;
- état d'ischémie cérébrale par souffrance cérébrale en cours d'endartériectomie carotidienne ou en cours de chirurgie nécessitant une circulation extracorporelle ;
- radiothérapie des tumeurs cérébrales (neuroprotection) ;
- traitement du mal aigü des montagnes;

Etude expérimentale

L'activité a été mise en évidence dans le test suivant, adapté de Singh, L. et Coll., Brit. J. Pharmacol., 99, 285 (1990).

**Crises induites par l'acide N-méthyl-DL-aspartique (NMDLA):**
le sel d'acide N-méthyl-DL-aspartique (NMDLA), à la concentration de 42,5 mg/cm3 est injecté par voie intra-veineuse (i.v.) (0,14 cm3/mn), dans la veine de la queue d'une souris (mâle CD1, Charles River), jusqu'à ce qu'une convulsion soit provoquée.

Cette convulsion représente le point final du test, et la souris est tuée immédiatement après l'apparition de cette convulsion. La durée entre l'injection du NMDLA et la crise est mesurée, et la dose seuil de NMDLA requise pour provoquer cette crise est calculée pour chaque souris.

Pour les antagonistes, les produits étudiés sont administrés par voie sous-cutanée, 30 minutes avant l'injection de NMDLA, à la dose seuil, nécessaire pour introduire une crise. Les résultats sont exprimés en pourcentage d'accroissememt du temps d'apparition de la crise, par comparaison avec le groupe contrôle (ayant reçu seulement le NMDLA), selon la formule suivante.

$$\frac{\text{dose seuil de NMDLA dans le groupe traité} \times 100}{\text{dose seuil de NMDLA dans le groupe contrôle}} - 100$$

La dose minimale active est la première dose significativement différente du contrôle qui correspond à une valeur du rapport ci-dessus supérieure à 20.

Il a été démontré que les doses actives se situent dans un intervalle, compris entre 1 et 3 mg/kg.

Les résultats obtenus figurent dans le tableau ci-après.

| Exemple N° | Structure | % d'augmentation du temps d'apparition des convulsions, à la dose de 3 mg/kg s.c. |
|---|---|---|
| 1 | (Série L) | 64 |
| 2 | (Série L) | 61 |

La préparation des produits a été décrite dans le brevet US 5 049 669.

La présente invention concerne l'obtention d'un médicament contenant au moins un produit mentionné ci-dessus éventuellement sous forme de sel, à l'état pur ou sous forme d'une composition pharmaceutique en association avec tout autre diluant ou adjuvant pharmaceutiquement acceptable et compatible.

Les compositions selon l'invention peuvent être utilisées par voie parentérale ou orale, à titre préventif et/ou curatif.

Les compositions stériles pour administration parentérale qui peuvent être notamment utilisées sous forme de perfusions sont de préférence des solutions aqueuses ou non aqueuses, des suspensions ou des émulsions.

Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles, qui peuvent être dissoutes au moment de l'emploi dans un milieu stérile injectable.

Comme compositions solides, pour administration orale, peuvent être utilisés des comprimés, des gélules, des poudres ou des granulés.

Dans ces compositions, le principe actif selon l'invention (éventuellement associé à un autre produit pharmaceutiquement compatible) est mélangé à un ou plusieurs diluants ou adjuvants inertes, tels que saccharose, lactose ou amidon.

Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que des diluants, par exemples des produits mouillants, édulcorants ou aromatisants.

En thérapeutique humaine, les produits selon l'invention sont particulièrement utiles dans le traitement ou la prévention des accidents vasculaires cérébral, hémorragique ou embolique, d'hémorragie sous-arachnoïdienne, traumatisme crânien avec souffrance cérébrale, état d'ischémie cérébrale par souffrance cérébrale en crises d'endartériéctomie carotidienne ou en cours de chirurgie nécessitant une circulation extra-corporelle, état de mal neuroleptique avec souffrance cérébrale, radiothérapie des tumeurs cérébrales (Neuroprotection), traitement du mal aigü des montagnes.

Les doses dépendent de l'effet recherché et de la durée du traitement.

Pour un adulte, elles sont généralement comprises entre 0,005 à 1 mg/kg. i.v, par jour.

D'une façon générale, le médecin déterminera la posologie qu'il estime la plus appropriée en fonction de l'âge, du poids et de tous les autres facteurs propres au sujet à traiter.

L'exemple suivant illustre une composition selon l'invention :

## Exemple :

On prépare selon la technique habituelle une solution pour administration intra-veineuse dosée à 25 mg/cm3 de produit actif (base) :

| | |
|---|---|
| - Chlorhydrate de N-n.propyl [(pyrrolidinyl-1)-1 propyl-2]-10 phénothiazinecarboxamide-2, série L | 2,70 g |
| - Acide ascorbique | 0,100 g |
| - Sulfite neutre de sodium | 0,050 g |
| - Soude 1N          environ | 0,08 cm$^3$ |
| - NaCl          environ | 0,650 g |
| - Eau pour préparation injectable          q.s.p | 100 cm$^3$ |

## Revendications

1. Application d'un dérivé de la phénothiazine, choisi parmi le N-n-propyl-[pyrrolidinyl-1)-1 propyl-2]-10 phénothiazinecarboxamide-2 (série L) et le N-(méthyl-2-butyl) [(pyrrolidinyl-1)-1-propyl-2]-10 phénothiazinecarboxamide-2 (série L) ainsi que ses sels pour obtenir un médicament destiné au traitement préventif et/ou curatif de l'ischémie et/ou de l'hypoxie.

## Claims

1. Application of a phenothiazine derivative chosen from N-n-propyl-10-[1-(1-pyrrolidinyl)-2-propyl]-2-phenothiazinecarboxamide (L series) and N-(2-methylbutyl)-10-[1-(1-pyrrolidinyl)-2-propyl]-2-phenothiazinecarboxamide (L series), as well as its salts, for obtaining a medicinal product intended for the preventive and/or curative treatment of ischaemia and/or hypoxia.

## Patentansprüche

1. Verwendung eines Derivats des Phenothiazins, ausgewählt unter N-n-Propyl-10-[1-(pyrrolidin-1-yl)-2-propyl]-phenothiazin-2-carboxamid (Reihe L) und N-(2-Methylbutyl)-10-[1-(pyrrolidin-1-yl)-2-propyl]-phenothiazin-2-carboxamid (Reihe L) sowie seiner Salze, um ein Arzneimittel zu erhalten, welches zur Vorbeugung und/oder Heilbehandlung der Ischämie und/oder der Hypoxie bestimmt ist.